# EUROPEAN PATENT SPECIFICATION

(11) **EP 1 459 739 B1**
(45) Date of publication and mention of the grant of the patent: **02.01.2008**
(21) Application number: 03006160.0
(22) Date of filing: 19.03.2003
(51) Int. Cl.: A61K 9/20

(54) **Non-hygroscopic pharmaceutical compositions containing non-hydrated quinoline carboxylic acids**
Nicht-hygroskopische pharmazeutische Zusammensetzungen die nicht hydratisierte Quinolon-Carbonsäure enthalten
Compositions pharmaceutiques non-hygroscopique contenant des acides carboxiliques quinilones non-hydratés

(43) Date of publication of application: 22.09.2004
(73) Proprietor: The Jordanian Pharmaceutical Manufacturing Co. Ltd., 11821 Amman (JO)
(72) Inventor: Badwan, Adnan, Dr, Amman 11185 (JO); Nabulsi, Lina, Najati, 11110 Amman (JO); Omari, Mahmoud, M, 11821 Amman (JO)
(74) Representative: Winkler, Andreas Fritz Ernst

(56) References cited:
- EP-A- 0 855 183
- WO-A-02/39992
- US-B1- 6 187 341
- BAUER; FRÖMMING; FÜHRER: 'Pharmazeutische Technologie 4. Auflage, Kapitel 14', 1997, GUSZTAV FISCHER VERLAG * page 292 - page 299 *
- HUNNIUS: 'Pharmazeutisches Wörterbuch, 8. Auflage', 1998, WALTER DE GRUYTER VERLAG * page 618 - page 620 *

## Description

### BACKGROUND OF THE INVENTION

The invention relates to the formulation of non-hydrated quinoline carboxylic acids with various additives to produce non-hygroscopic formulas for oral dosage forms.

Said compounds are mainly hygroscopic and can form different hydrates. For example, norfloxacin in its anhydrous form suffers from water uptake in its pure state or in solid dosage forms. It can absorb water to produce hydrates with different molar ratios (sesquihydrate, dihydrate). The water percentages are 7.5% and 10% for sesquihydrate and dihydrate, respectively.

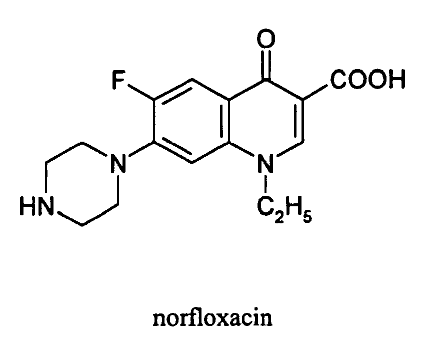

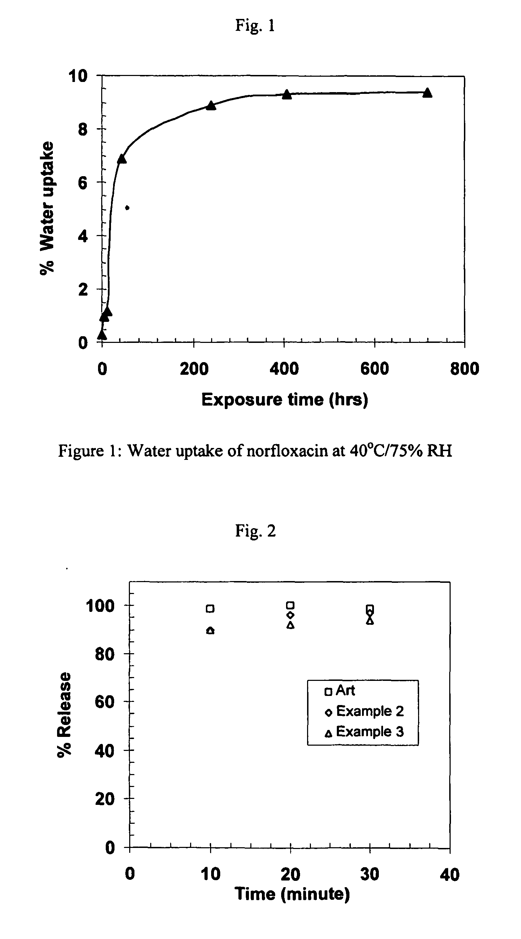

The water uptake for norfloxacin and its formulations may reach up to 30% and more depending on the manufacturing procedures (wet or dry granulation), and storage conditions of temperature and humidity (see Fig. 1).

In Italian patent application 20764A/79, water (about 2% to about 15%) is included in formulations for tablet oral dosage forms. From the manufacturing process and control point of views it is not favorable to have a significant variation in water content. Also, formulation of a hygroscopic compound such as norfloxacin with low water content is liable to accommodate more water. These types of formulations may require a high protective package such as aluminum/ aluminum.

WO0239992 teaches adducts of natural polysaccharide polymers with quinolinic active agents. The adducts, containing norflaxin, maltodextrin and hydrochoric acid are first dissolved in aqueous solution and then dried to yield a powder

A direct compression tablets formulation for quinoline carboxylic acids with low water content (< 2%) is disclosed in European patent application EP 189 114. In this patent application, the formulation contains an antibacterial agent, preferably norfloxacin, and minimal amounts of disintegrant, filler/ binder and lubricant. Such formulations require a high protective package such as aluminum/ aluminum. In addition, direct compression may be considered as a critical manufacturing process, especially for formulations containing high amounts of the active ingredient such as norfloxacin (e.g. 400 mg per tablet). As a result, control of physical properties of the active ingredient such as the particle size distribution is required.

### SUMARY OF THE INVENTION

In this invention, different formulas of non-hydrated quinoline carboxylic acids are disclosed produced by using wet granulation with water. The obtained formulas have low water content (< 3%) and are non-hygroscopic.

A wet granulation tablet formulation has been discovered where water is included in a granulation step, followed by drying to obtain granules of low water content (< 3%) and being non-hygroscopic compared with prior art formulations, while maintaining equivalent characteristics (dissolution, disintegration, bioavailability and physical properties) of the tablet prepared therefrom. The wet granulation formulation may be applied for other quinoline carboxylic acids.

### DESCRIPTION OF THE INVENTION

This invention covers a wet granulation formulation of quinoline carboxylic acid antibacterial agents.

Norfloxacin, ciprofloxacin, ofloxacin, enrofloxacin, lemofloxacin, levofloxacin, enoxacin, pefloxacin, balofloxacin, clinafloxacin, difloxacin, fleroxacin, grepafloxacin, gatifloxacin and the like are examples of useful quinoline carboxylic acids. A preferred quinoline carboxylic acid is norfloxacin.

The present formulation involves granulating of said antibacterial agent with a stabilizer, preferably in the presence of processing aids. The processing aids may include a filler, a disintegrant, a binder and/or a lubricant.

The stabilizer is selected from inorganic acids (e.g. hydrochloric, sulfuric or phosphoric acid) or organic acids (e.g. anhydrous citric, hydrate citric, fumaric, malic, maleic, tartaric, glutaric, benzenesulfonic, benzoic or salicylic acid).

The amount of stabilizer is 10-35% wt/wt, more favorable 20-35% wt/wt and the optimal is 35% wt/wt.

The present wet formulation includes granulation of an antibacterial agent with the stabilizer dissolved in water, ethanol or a mixture of water/ethanol (10/90 to 90/10 v/v). The obtained granules have a water content less than 2-3% after drying at a temperature range of 55°C-65°C. Then a filler, a disintegrant and a lubricant are added followed by compression to produce tablet. The tablet is film coated using a known aqueous coating system. A modified cellulose e.g. hydroxypropylcellulose and/or hydroxypropylmethylcellulose may, for example, be used as film former.

The following examples illustrate tablet formulations containing 400 mg of an antibacterial agent (norfloxacin).

### Example 1 (Comparative example)

| **No** | **Component** | **Function** | **Mg/tablet** | **Weight %** |
|---|---|---|---|---|
| 1 | Norfloxacin | Active ingredient | 400 | 80 |
| 2 | Microcrystalline cellulose | Filler | 85.5 | 17 |
| 3 | Croscarmellose sodium | Disintegrant | 10 | 2 |
| 4 | Magnesium stearate | Lubricant | 4.5 | 1 |

### Example 2 (Formulation of invention)

| **No** | **Component** | **Function** | **mg/tablet** | **Weight %** |
|---|---|---|---|---|
| 1 | Norfloxacin | Active ingredient | 400 | 63.5 |
| 2 | Anhydrous citric acid | Stabilizer | 200 | 31.7 |
| 3 | Sodium starch glycolate | Disintegrant | 23 | 3.7 |
| 4 | Magnesium stearate | Lubricant | 7 | 1.1 |

The tablet preparation is carried out by granulating the antibacterial agent (norfloxacin) with water in the absence of stabilizer (example 1) and in the presence of stabilizer (examples 2 and 3). Example 3 is the same as in example 2, but a mixture of water/ethanol (50/50, v/v) is used instead of water. The amount of stabilizer represents about 50% wt/v compared to granulation solvent (i.e. 200 mg citric acid dissolved in 400 ml water). Drying at a temperature of 60°C to obtain suitable granules is carried out. Sizing of granules is carried out followed by addition of a filler, a disintegrant and a lubricant.

The water contents of the powder obtained in example 1 and 2 are 12% and 2.2%, respectively. This indicates that the presence of stabilizer prevents the active ingredient (norfloxacin) to form hydrates. In addition, incubation of powder obtained in example 2 at 40°C for 3 months in an open container do not show any significant increase in water content.

The tablet preparation is carried out by granulating an antibacterial agent (norfloxacin) with stabilizer dissolved in a suitable amount of water or a mixture of water/ethanol (50/50, v/v). The amounts of stabilizer represent about 10-35%. The amount of stabilizer represents about 50% wt/v compared to granulation solvent (i.e. 200 mg citric acid dissolved in 400 ml water). Both granulation solvents may be used, but a mixture of water/ethanol is more applicable. Drying to obtain suitable granules with a water content of less than 3% is carried out at a temperature range of 55°C-65°C. Sizing of granules is carried out followed by addition of a filler, a disintegrant and a lubricant. A filler such as microcrystalline cellulose may enhance the powder flowability. The final mix is compressed to produce tablets. The tablet may be film coated using a water-based system.

Stability study of norfloxacin in the presence of stabilizer is monitored by chromatographic methods (USP 25,2002, under norfloxacin and norfloxacin tablets). The stability results at 40°C/75%RH for 3 months in open and closed containers shows no sign of instability.

The present invention produces a non-hygroscopic formulation although wet granulation in the presence of water is used. This provides process advantages such as using low water protective packaging material such as PVC, reproducible formulas with a water content of less than 3%, less sensitive as wet granulation for the variation of norfloxacin particle sizes, and low cost process.

The following table represents the physical properties of tablets containing 400 mg norfloxacin obtained from EP 189 114 and according to the present formulation.

| **Tablet formulation** | **Disintegration time** | **Hardness** | **Dissolution¹** | | |
|---|---|---|---|---|---|
| | **(min)** | **(N)** | **(%)** | | |
| | | | **10 min** | **20 min** | **30 min** |
| EP 189 114 | < 5 | 164-182 | 99 | 100 | 99 |
| Example 2 | < 5 | 94-105 | 90 | 96 | 97 |
| (water)* | | | | | |
| Example 3 | < 5 | 124-140 | 90 | 92 | 94 |
| (50% ethanol)* | | | | | |

| | | | | | |
|---|---|---|---|---|---|
| * Granulation solvent, ¹ using USP method under Norfloxacin Tablets | | | | | |

The data as shown in the above table and Fig. 2 indicate that both formulations of the EP 189 114 and the present patent produce tablets of fast disintegration and dissolution profiles. Both formulations are substantially equivalent.

Tablets containing more or less than 400 mg can be prepared as needed.

The features disclosed in the foregoing description, in the claims and/or the accompanying drawings may both separately and in any combination thereof be material for realising the invention in diverse forms thereof.

## Claims

1. A wet granulation formulation containing at least one quinoline carboxylic acid antibacterial agent, a stabilizer selected from the group consisting of inorganic acids or organic acids, and less than 3 % water.

2. The wet granulation formulation according to claim 1, wherein the amount of the stabilizer is 10 to 35 % wt/wt.

3. The wet granulation formulation according to claim 1 or 2, wherein the stabilizer is selected from the group consisting of hydrochloric, sulphuric or phosphoric acid, or anhydrous citric, hydrated citric, fumaric, malic, maleic, tartaric, glutaric, benzensulfonic, benzoic or salicylic acid.

4. The wet granulation formulation according to claim 3, wherein the stabilizer is anhydrous citric acid.

5. The wet granulation formulation according to any of claims 1 to 4, wherein the quinoline carboxylic acid is norfloxacin.

6. The wet granulation formulation according to claim 5, wherein the amount of norfloxacin is 60-75 % wt/wt, and the content of stabilizer is 10-35% wt/wt.

7. Use of the wet granulation formulation according to any of claims 1 to 6 for preparing an oral dosage form.

8. A tablet comprising a wet granulation formulation according to any of claims 1 to 6.

9. The tablet according to claim 8, wherein said tablet further comprises a filler/a binder, a disintegrant and/or a lubricant.

10. The tablet according to claim 9, wherein said tablet comprises about 60-75 % norfloxacin, 10-35 % of a stabilizer, 5-15% of a binder/filler, 1-5% of a disintegrant and/or 0.5-2% of a lubricant.

11. The tablet according to any of claims 8 to 10, wherein said tablet contains less than 3% water.

12. The tablet according to any of claims 8 to 11, wherein said tablet has a conventional film coating.

13. A method for preparing a wet granulation formulation according to any of claims 1to 6, comprising the steps:
a) granulating at least one quinoline carboxylic acid antibacterial agent and at least one stabilizer selected from the group consisting of inorganic acids or organic acids, wherein the stabilizer is dissolved in water, ethanol or a mixture of water/ethanol (10/90 to 90/10 v/v), and
b) drying the obtained granulate to a water content of less than 3%.

14. Method according to claim 13, wherein wet granulated composition is dried at a temperature range of 55°C - 65°C.

15. A method for preparing a tablet according to any of claims 8 to 12 comprising the steps a) and b) according to claim 13,
c) mixing the dried granulate with excipients; and
d) pressing the mixture into tablet form.

16. Method according to claims 15, wherein the excipients are a filler/a binder, a disintegrant and/or a lubricant.

## Patentansprüche

1. Nassgranulierungsformulierung, die mindestens eine Chinolincarbonsäure als antibakterielles Mittel, einen Stabilisator, der ausgewählt ist aus der Gruppe, die aus anorganischen Säuren oder organischen Säuren besteht, und weniger als 3% Wasser enthält.

2. Nassgranulierungsformulierung nach Anspruch 1, wobei die Menge an Stabilisator 10 bis 35% Gew./Gew. beträgt.

3. Nassgranulierungsformulierung nach Anspruch 1 oder 2, wobei der Stabilisator ausgewählt ist aus der Gruppe, die aus Salz-, Schwefel- oder Phosphorsäure oder wasserfreier Zitronensäure, hydratisierter Zitronen-, Fumar-, Äpfel-, Malein-, Wein-, Glutar-, Benzolsulfon-, Benzoe- oder Salicylsäure besteht.

4. Nassgranulierungsformulierung nach Anspruch 3, wobei der Stabilisator wasserfreie Zitronensäure ist.

5. Nassgranulierungsformulierung nach einem der Ansprüche 1 bis 4, wobei die Chinolincarbonsäure Norfloxacin ist.

6. Nassgranulierungsformulierung nach Anspruch 5, wobei die Menge an Norfloxacin 60 bis 75% Gew./Gew. und der Gehalt an Stabilisator 10 bis 35 Gew./Gew. beträgt.

7. Verwendung der Nassgranulierungsformulierung nach einem der Ansprüche 1 bis 6 zum Herstellen einer oralen Dosierform.

8. Tablette, welche eine Nassgranulierungsformulierung nach einem der Ansprüche 1 bis 6 umfasst.

9. Tablette nach Anspruch 8, wobei die Tablette weiterhin ein Füllmittel/ein Bindemittel, ein Sprengmittel und/oder ein Gleitmittel umfasst.

10. Tablette nach Anspruch 9, wobei die Tablette ungefähr 60 bis 75% Norfloxacin, 10 bis 35% eines Stabilisators, 5 bis 15 % eines Bindemittels/Füllmittels, 1 bis 5% eines Sprengmittels und/oder 0,5 bis 2% eines Gleitmittels umfasst.

11. Tablette nach einem der Ansprüche 8 bis 10, wobei die Tablette weniger als 3% Wasser enthält.

12. Tablette nach einem der Ansprüche 8 bis 11, wobei die Tablette eine herkömmliche Filmbeschichtung aufweist.

13. Verfahren zum Herstellen einer Nassgranulierungsformulierung nach einem der Ansprüche 1 bis 6, das folgende Schritte umfasst:
(a) Granulieren von mindestens einer Chinolincarbonsäure als antibakteriellem Mittel und mindestens einem Stabilisator, der ausgewählt ist aus der Gruppe, die aus anorganischen Säuren oder organischen Säuren besteht, wobei der Stabilisator in Wasser, Ethanol oder einer Mischung aus Wasser/Ethanol (10/90 bis 90/10 Vol./Vol.) gelöst ist, und
(b) Trocknen des erhaltenen Granulats zu einem Wassergehalt von weniger als 3%.

14. Verfahren nach Anspruch 13, wobei die nassgranulierte Zusammensetzung bei einer Temperatur im Bereich von 55°C bis 65°C getrocknet wird.

15. Verfahren zum Herstellen einer Tablette nach einen der Ansprüche 8 bis 12, das die Schritte (a) und (b) nach Anspruch 13 umfasst,
(c) Mischen des getrockneten Granulats mit Hilfsstoffen; und
(d) Pressen der Mischung in Tablettenform

16. Verfahren nach Anspruch 15, wobei die Hilfsstoffe ein Füllmittel/ein Bindemittel, ein Sprengmittel und/oder ein Gleitmittel sind.

## Revendications

1. Formulation pour granulation humide contenant au moins un agent antibactérien acide quinoléine carboxylique, un stabilisant sélectionné dans le groupe constitué par des acides inorganiques ou organiques, et moins de 3% d'eau.

2. Formulation pour granulation humide selon la revendication 1, dans laquelle la quantité de stabilisant est de 10 à 35% en poids.

3. Formulation pour granulation humide selon la revendication 1 ou 2, dans laquelle le stabilisant est sélectionné dans le groupe constitué par l'acide chlorhydrique, sulfurique ou phosphorique, ou l'acide citrique anhydre, l'acide citrique hydraté, l'acide fumarique, malique, maléique, tartrique, glutarique, benzènesulfonique, benzoïque ou salicylique.

4. Formulation pour granulation humide selon la revendication 3, dans laquelle le stabilisant est l'acide citrique anhydre.

5. Formulation pour granulation humide selon l'une quelconque des revendications 1 à 4, dans laquelle l'acide quinoléine carboxylique est la norfloxacine.

6. Formulation pour granulation humide selon la revendication 5, dans laquelle la quantité de norfloxacine est de 60-75% en poids et la teneur en stabilisant est de 10-35% en poids.

7. Utilisation de la formulation pour granulation humide selon l'une quelconque des revendications 1 à 6, pour préparer une forme galénique orale.

8. Comprimé comprenant une formulation pour granulation humide selon l'une quelconque des revendications 1 à 6.

9. Comprimé selon la revendication 8, dans lequel ledit comprimé comprend en outre un agent de remplissage/liant, un agent de délitement et/ou un lubrifiant.

10. Comprimé selon la revendication 9, dans lequel ledit comprimé comprend environ 60-75% de norfloxacine, 10-35% d'un stabilisant, 5-15% d'un agent de remplissage/liant, 1-5% d'un agent de délitement et/ou 0,5-2% d'un lubrifiant.

11. Comprimé selon l'une quelconque des revendications 8 à 10, dans lequel ledit comprimé contient moins de 3% d'eau.

12. Comprimé selon l'une quelconque des revendications 8 à 11, dans lequel ledit comprimé a un pelliculage classique.

13. Procédé pour préparation d'une formulation pour granulation humide selon l'une quelconque des revendications 1 à 6, comprenant les étapes suivantes :
a) granulation d'au moins un agent antibactérien de type acide quinoléine carboxylique et d'au moins un stabilisant sélectionné dans le groupe constitué par des acides inorganiques ou organiques, dans laquelle le stabilisant est dissous dans l'eau, l'éthanol ou un mélange eau/éthanol (10/90 à 90/10 en volume), et
b) séchage du granulé obtenu jusqu'à une teneur en eau inférieure à 3%.

14. Procédé selon la revendication 13, dans lequel la composition granulée humide est séchée à une température comprise entre 55°C et 65°C.

15. Procédé pour préparer un comprimé selon l'une quelconque des revendications 8 à 12, comprenant les étapes a) et b) selon la revendication 13,
c) le mélange du granulé séché avec les excipients, et
d) le pressage du mélange en comprimés.

16. Procédé selon la revendication 15, dans lequel les excipients sont un agent de remplissage/liant, un agent de délitement et/ou un lubrifiant.
